# EUROPEAN PATENT SPECIFICATION

(11) **EP 2 622 326 B1**
(45) Date of publication and mention of the grant of the patent: **30.07.2014**
(21) Application number: 11784577.6
(22) Date of filing: 29.08.2011
(51) Int. Cl.: G01N 21/65, G01N 33/58, G01N 33/543, B82Y 15/00, B82Y 30/00

(54) **METHOD FOR ACTIVATION OF AQUEOUS SILVER NANOPARTICLE DISPERSIONS FOR SURFACE ENHANCED RAMAN SPECTROSCOPY**
VERFAHREN ZUR AKTIVIERUNG VON WÄSSRIGEN SILBERNANOPARTIKELDISPERSIONEN FÜR OBERFLÄCHENVERSTÄRKTE RAMAN-SPEKTROSKOPIE
PROCÉDÉ D'ACTIVATION DE DISPERSIONS AQUEUSES DE NANOPARTICULES D'ARGENT DANS LA SPECTROSCOPIE RAMAN EXALTÉE DE SURFACE

(30) Priority: 29.09.2010 CZ 20100708
(43) Date of publication of application: 07.08.2013
(73) Proprietor: Univerzita Palackého v Olomouci, 771 47 Olomouc (CZ)
(72) Inventor: PRUCEK, Robert, 783 22 Olbramice (CZ); KVITEK, Libor, 783 72 Velky Tynec (CZ); PANACEK, Ales, 783 91 Unicov (CZ); RANC, Vaclav, 783 16 Dolany (CZ); ZBORIL, Radek, 779 00 Olomouc (CZ)
(74) Representative: Hartvichova, Katerina
(86) International application number: PCT/CZ2011/000083
(87) International publication number: WO 2012/041262

(56) References cited:
- KVITEK L ET AL: "The influence of complexing agent concentration on particle size in the process of SERS active silver colloid synthesis", JOURNAL OF MATERIALS CHEMISTRY R. SOC. CHEM. UK, vol. 15, no. 10, 14 March 2005 (2005-03-14), pages 1099-1105, XP055015704, ISSN: 0959-9428
- N.R. JANA ET AL: "Anisotropic Metal Nanoparticles for Use as Surface-Enhanced Raman Substrates", ADVANCED MATERIALS, vol. 19, no. 13, 2 July 2007 (2007-07-02), pages 1761-1765, XP55015806, ISSN: 0935-9648, DOI: 10.1002/adma.200601749
- KEN-TYE YONG ET AL: "Templated Synthesis of Gold Nanorods (NRs): The Effects of Cosurfactants and Electrolytes on the Shape and Optical Properties", TOPICS IN CATALYSIS, KLUWER ACADEMIC PUBLISHERS-PLENUM PUBLISHERS, NE, vol. 47, no. 1-2, 6 February 2008 (2008-02-06), pages 49-60, XP019581515, ISSN: 1572-9028
- JANINA KNEIPP ET AL: "Surface-enhanced Raman scattering: a new optical probe in molecular biophysics and biomedicine", THEORETICAL CHEMISTRY ACCOUNTS ; THEORY, COMPUTATION, AND MODELING, SPRINGER, BERLIN, DE, vol. 125, no. 3-6, 13 November 2009 (2009-11-13), pages 319-327, XP019777551, ISSN: 1432-2234
- PRUCEK R ET AL: "Re-crystallization of silver nanoparticles in a highly concentrated NaCl environment -a new substrate for surface enhanced IR-visible Raman spectroscopy", CRYSTENGCOMM, vol. 13, 21 January 2011 (2011-01-21), pages 2242-2248, XP009154348, online

## Description

### Field of Art

The present invention relates to a method for activation of aqueous silver nanoparticle dispersions for purposes of surface enhanced Raman spectroscopy, which represents a very promising and sensitive analytical method.

### Background Art

The discovery of surface enhanced Raman scattering (SERS) on a silver electrode by Fleischmann in 1974, and especially its re-discovery on colloidal silver particles by Creighton in 1977 started the extensive development of a new and very sensitive analytical method that allows the detection of molecules in the concentration range from pico- to femtomols (Doerning W. E. and Nie S. M., J Phys Chem B, 106, 2002). A high enhancement of SERS, reaching the values of up to 10¹⁵, even allowed the detection of individual molecule adsorbed on a single silver nanoparticle (Michaels M. et al. J Am Chem Soc, 121, 1999; Nie S. and Emory S. R., Science, 275, 1997). Some studies have shown that so high a value of the enhancement can be achieved only on particles of a certain sizes which are referred to as "hot particles". These particles' optimum size depends on the wavelength of the laser used for the excitation and ranges approximately from 70 nm to 200 nm for the excitation wavelengths in the range from 488 nm to 647 nm (Emory S. R.et al., J Am Chem Soc 120, 1998). For the commonly used argon lasers (wavelength of 514.5 nm), silver particles of the size of about 90 nm are the most effective ones. Based on the mentioned dependence of the size of the "hot particles" for a given laser wavelength, it can be expected that when using lasers in the red (785 nm) and near-infrared area (1064 nm), the maximum enhancement of Raman signal should be obtained on silver particles of the size of around 400 nm. Unfortunately, particles of this dimension are unstable in solution and they usually sediment within a few hours. Silver nanoparticles of the size ranging from units of nanometers to tens of nanometers can be stable for several months or years, even without any extra stabilization. However, these small particles themselves usually do not provide surface enhancement of the Raman signal. For this purpose, they must be activated for example by the addition of some inorganic ion solution. The most frequently used activation agents of the silver nanoparticles include halide ions, particularly chlorides. However, the mechanism of the activation of these ions is not yet fully explained; one of possible explanations of this effect is based on a partial aggregation of silver nanoparticles caused by mentioned ions. The state-of-art procedures of silver nanoparticle activation by means of chloride ion solutions employ the final concentrations in the range of from 0.001 mol.dm⁻³ to 0.02 mol.dm⁻³ (Leopold, N. and Lendl, B. J Phys Chem B 107, 2003; Michaels A. et al., J Am Chem Soc 121, 1999; Leng W. N. et al., J Raman Spectrosc 37, 2006; Zhang P. X. et al., J Raman Spectrosc 21, 1990; Campbell M. et al., J Raman Spectrosc 30, 1999; Doering W. E. and Nie S. M., J Phys Chem B 106, 2002). But in these cases, a slow aggregation of silver nanoparticles occurs, resulting in Raman signal irreproducibility and often also in a long activation time.

### Disclosure of the Invention

The object of the invention is a method for a fast activation of aqueous dispersions of silver nanoparticles for purposes of surface enhanced Roman spectroscopy, wherein a solution of chloride ions is added to the aqueous dispersion of silver nanoparticles in the presence of oxygen in such an amount that the final chloride ion concentration in the activated dispersion ranges from 0.4 mol.dm⁻³ to 1 mol.dm⁻³.

The advantage of the method of activation according to the present invention is that it allows to prepare silver particles suitable also for the application in surface enhanced Raman spectroscopy that uses lasers with the wavelength in a near-infrared area.

In case of the method according to the present invention, gradual recrystallization of nanoparticles occurs. After about 15 minutes following the addition of the chloride anions with the final concentration of 0.4 mol.dm⁻³, particles of the size of about 400 nm are already present in the activated dispersion. This particle size modification process caused by the recrystallization is reproducible. Hence another advantage of the present invention is that it allows a fast and reproducible activation of silver nanoparticles for the purposes of SERS through their recrystallization, which is different from the state of the art methods which lead to a slow and often irreproducible aggregation of silver particles.

It is an aspect of the invention that the re-crystallization and activation occurs in the presence of oxygen. The presence of oxygen in the activated dispersion is an important requirement for the nanoparticle recrystallization and activation. Usually, the amount of oxygen naturally dissolved in water under standard conditions (ambient temperature, atmospheric pressure) is sufficient for a successful course of the activation. Where necessary, more oxygen can be added into the dispersion during the activation. The adding of oxygen can be performed, e.g., by several short shakings of the dispersion during the activation, by a vigorous agitation, by adding oxygen from a container, by chemically preparing oxygen (e.g., using a chemical source of oxygen) or by any other suitable method. In total absence of oxygen in the activated dispersion, large aggregates of the initial nanoparticles are formed that do not manifest any surface enhancement of Raman signal.

When using a lower final chloride ion concentrations in the activated dispersion (about 0.1 mol.dm⁻³), also in the presence of oxygen, the activation and recrystallization process is not as fast and reproducible as in the case of the higher final chloride anion concentrations. Moreover, in these cases the particles recrystallize only partially within the same time period. For that reason, it is advantageous to use higher final chloride ion concentrations, i.e. 0.4 mol.dm⁻³ and higher.

The final chloride ion concentration in the activated silver nanoparticle dispersion is in the range of from 0.4 mol.d⁻³ to 1 mol.dm⁻³, more preferably 0.4 to 0.8 mol.dm⁻³

For the activation of silver nanoparticles for the spectroscopy using a laser with the wavelength of 1064 nm, it is appropriate to use higher final chloride ion concentrations in the final activated dispersion, particularly the concentrations of from 0.4 mol.dm⁻³ to 0.8 mol.dm⁻³.

The concentration of chloride ions in the chloride ion stock solutions used for the activation is preferably in the range of from 0.125 mol.dm⁻³ to saturated solution concentration, more preferably 1 mol.dm⁻³ to saturated solution concentration, most preferably 1 mol.dm⁻³ to 4 mol.dm⁻³.

Any water-soluble salt containing the chloride anion can be used as the source of chloride ions, provided that its stock solution can be prepared with a sufficient concentration, i.e. if the concentration of the chloride ions in the saturated solution thereof is sufficient for achieving the required final chloride ion concentration. Also, the cation contained in that salt should not interact with the activated silver nanoparticle dispersion. For example, chlorides of alkali metals (i.e. metals from the IA group of the periodic table of elements) and ammonium chloride are suitable salts.

The initial size of silver nanoparticles in the dispersion before the activation is typically in the range of from 10 nm to 100 nm.

The final Ag concentration in the activated silver nanoparticle dispersion is typically in the range of from 10⁻⁷ mol.dm⁻³ to 0.05 mol.dm⁻³, preferably 10⁻⁶ mol.dm⁻³ to 0.01 mol.dm⁻³, more preferably 10⁻⁴ mol.dm⁻³ to 10⁻³ mol.dm⁻³.

### Brief Description of Drawings

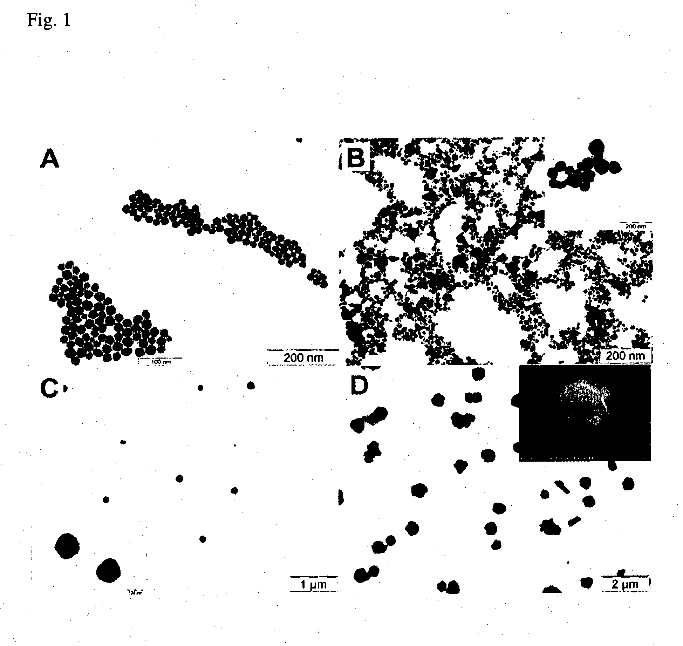

Figure 1 shows the transmission electron microscopy images of the primary silver nanoparticles and the transformed silver particles formed during 15 minutes after the addition of the sodium chloride solution with the concentration of 4 mol.dm⁻³ (the final concentration of 0.4 mol.dm⁻³). (A) primary silver nanoparticles with the size of approx. 20 nm; (B) silver particles formed after 30 seconds; (C) after 2 minutes; and (D) after 15 minutes following the addition of NaCl solution with the concentration of 4 mol.dm⁻³.

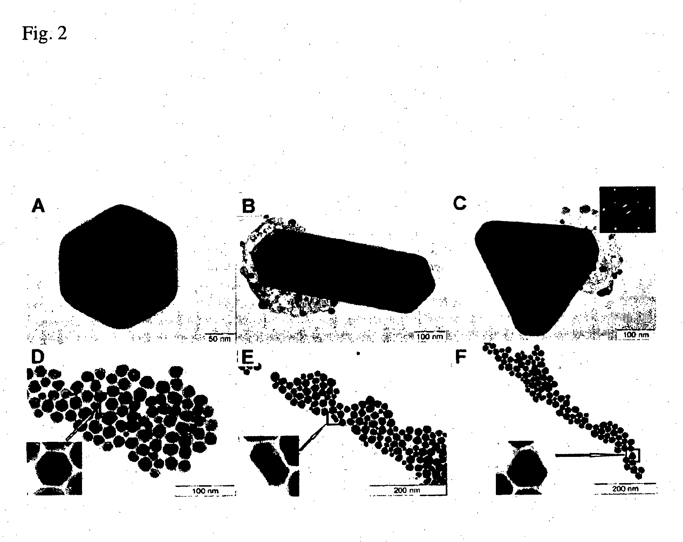

Figure 2 represents transmission electron microscopy images of recrystallized particles (A, B, C) with various morphologies of the size of several hundred nanometers formed 15 minutes after the addition of sodium chloride solution (the final concentration of 0.4 mol.dm⁻³) and transmission electron microscopy images of primary non-activated silver nanoparticles (D, E, F) with the same crystal shapes (see insets) before the addition of sodium chloride solution.

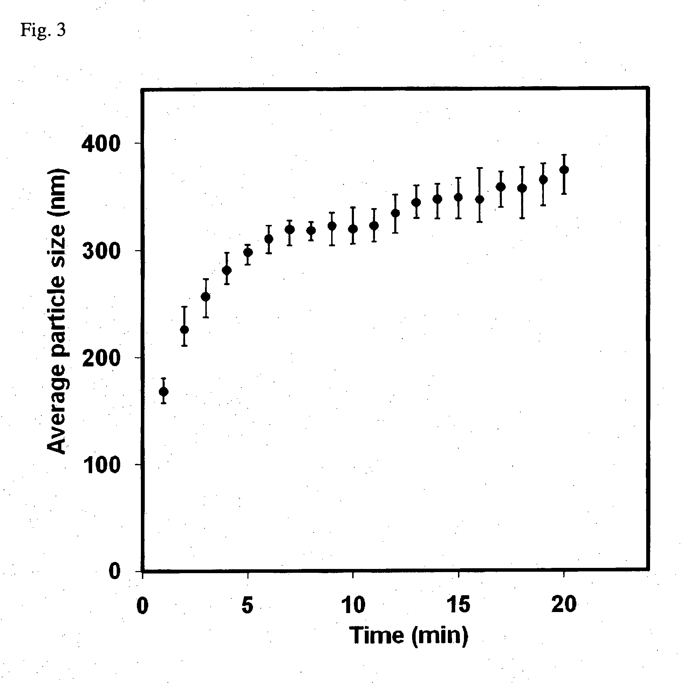

Figure 3 shows the time-dependent change of the average size of silver particles recorded during 20 minutes after the addition of 4M sodium chloride solution (the final concentration of 0.4 mol.dm⁻³). The data presented in the chart are an average obtained from 12 independent measurements performed during one year, wherein the error bars represent maximum and minimum determined values of silver particle average sizes. The average particle sizes were determined by DLS method.

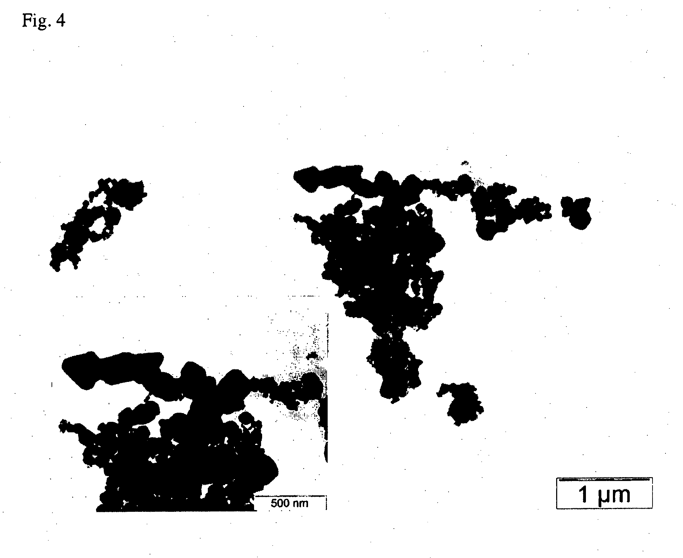

Figure 4 shows transmission electron microscopy images of silver nanoparticles of the initial size of approx. 20 nm transformed during 15 minutes following the addition of the sodium chloride solution with the concentration of 1 mol.dm⁻³ (the final concentration of 0.1 mol.dm⁻³).

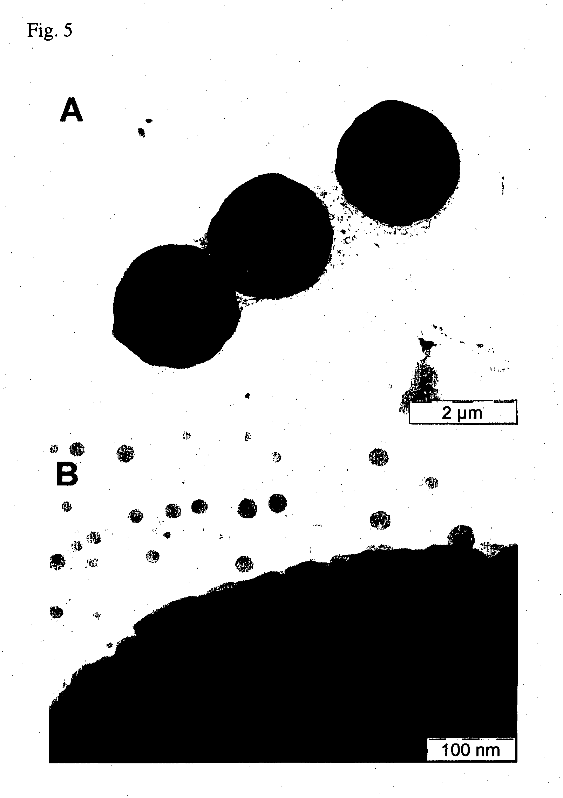

Figure 5 shows (A) transmission electron microscopy images of silver particle aggregates obtained after 15 minutes following the addition of sodium chloride solution (the final concentration of 0.4 mol.dm⁻³) to particles of the size of approx. 20 nm in the absence of oxygen in the activated dispersion, and (B) the detail of these aggregates' surface.

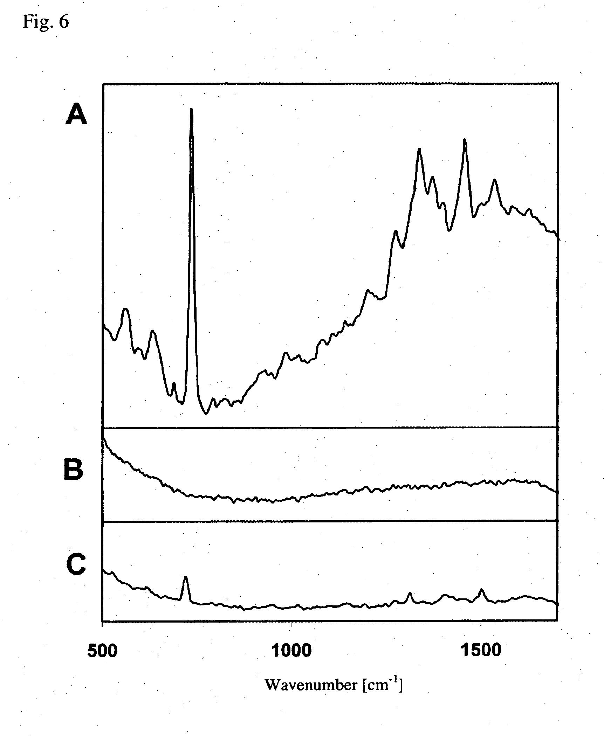

Figure 6 shows Raman spectra and surface enhanced Raman spectra of adenine recorded using a laser with the wavelength of 1064 nm. (A) Surface enhanced Raman spectrum of adenine with the concentration of 10⁻⁵ mol.dm⁻³ recorded in the presence of silver particles transformed by means of sodium chloride solution with the final concentration of 0.4 mol.dm⁻³. (B) Raman spectrum of adenine with the concentration of 10⁻⁵ mol.dm⁻³ recorded using silver nanoparticles of the size approx. 20 nm without the sodium chloride solution addition. (C) Raman spectrum of adenine of concentration equal to 10⁻¹ mol.dm⁻³.

### Examples of Carrying Out the Invention

Sodium chloride was obtained from Sigma-Aldrich. Silver nanoparticles were prepared by means of reduction of silver ion ammonium complex with maltose, glucose, ascorbic acid and sodium tetrahydridoborate (Kvitek et al., J Mater Chem 15, 2005, Panacek et al., J Phys Chem B 110, 2006, Creighton et al., J Chem Soc Faraday Trans II 75, 1979). The starting compounds were obtained from Sigma-Aldrich.

The size of nanoparticles was measured by Zetasizer Nano-ZS (Malvern, UK) instrument using the DLS (Dynamic Light Scattering) principle. The prepared dispersions were also characterized by UV-Vis absorption spectroscopy using Specord S600 spectrophotometer (Analytic Jena AG, Germany). The size of silver nanoparticles and their polydispersity was also verified by transmission electron microscopy (TEM) by JEM 2010 instrument (Jeol, Japan) using the accelerating voltage of 160 kV. The surface enhanced Raman spectra were measured on Jobin-Yvon T 64 000 instruments with the excitation wavelengths of 488 nm and 514.5 nm (Jobin-Yvon, France), HORIBA iHR550 with the excitation wavelength of 532 nm, Thermo Scientific DXR Microscope with the excitation wavelengths of 633 nm and 780 nm (Thermo Scientific, USA) and Nicolet FT-IR 6700 (Nicolet, USA) equipped with Raman module (NXR FT-Nicolet, USA) with the excitation wavelength of 1064 nm.

### Example 1

### Recrystallization of silver nanoparticles with an average size of 25 nm using sodium chloride solution with a concentration of 4 mol.dm⁻³

0.2 ml of a dispersion of silver nanoparticles with the average size of 25 nm and Ag concentration of 0.001 mol.dm⁻³ in the dispersion was transferred into a 3 ml cuvette. Then 0.7 ml of distilled water was added. After stirring the mixture, 0.1 ml of NaCl solution with the concentration of 4 mol.dm⁻³ was added. The final concentration of sodium chloride in the solution was 0.4 mol.dcm⁻³. The mixture was vigorously stirred for 10 seconds and then the changes in the silver particle dispersion characteristics were measured using the following methods: DLS, UV-Vis absorption spectroscopy, and TEM. After 15 minutes following the addition of chloride anions, particles with the size of 400 nm were already present in the activated dispersion (Fig. 1, 2). The process of the particle size change caused by the recrystallization is reproducible, as it was shown in the course of 12 measurements carried out during one year (Fig. 3).

### Example 2 (Comparative example)

### Transformation of silver nanoparticles with an average size of 25 nm using sodium chloride solution with a concentration of 1 mol.dm⁻³

0.2 ml of a dispersion of silver nanoparticles with the average size of 25 nm and Ag concentration of 0.001 mol.dm⁻³ in the dispersion was transferred into a 3 ml cuvette. Then 0.7 ml of distilled water was added. After stirring the mixture, 0.1 ml of NaCl solution with the concentration of 1 mol.dm⁻³ was added. The final concentration of sodium chloride in the solution was 0.1 mol.dm⁻³. The mixture was vigorously stirred for 10 seconds and then the changes of the silver particle dispersion characteristics were measured using the following methods: DLS, UV-Vis absorption spectroscopy, and TEM. When the final chloride ion concentration in the activated dispersion is 0.1 mol.dm⁻³, the particles recrystallize only partially and incompletely within the same time as in Example 1 (Fig. 4).

### Example 3

### Transformation of silver nanoparticles with an average size of 25 nm using sodium chloride solution with a concentration of 4 mol.dm⁻³ in the absence of oxygen in reaction solutions

0.2 ml of a dispersion of silver nanoparticles with the average size of 25 nm and Ag concentration of 0.001 mol.dm⁻³ in the dispersion was transferred into a 3 ml cuvette. Then 0.7 ml of distilled water was added. After stirring the mixture, 0.1 ml of NaCl solution with the concentration of 4 mol.dm⁻³ was added. The final concentration of sodium chloride in the solution was 0.4 mol.dm⁻³. All air oxygen has been removed from all solutions by a thorough bubbling with argon and the transformation was carried out in the absence of air. In this case, the primary particle recrystallization did not occur, but only their aggregation. On TEM images, we can observe aggregates of the size of several units of micrometers (Fig. 5); these aggregates prepared in the absence of oxygen are not suitable for use in the SERS.

### Example 4

### Recrystallization of silver nanoparticles with an average size of 25 nm using sodium chloride solution with a concentration of 4 mol.dm⁻³

0.1 ml of a dispersion of silver nanoparticles with the average size of 25 nm and Ag concentration of 0.001 mol.dm⁻³ in the dispersion was transferred into a 3 ml cuvette. Then 0.8 ml of distilled water was added. After stirring the mixture, 0.1 ml of NaCl solution with the concentration of 4 mol.dm⁻³ was added. The final concentration of sodium chloride in the solution was 0.4 mol.dm⁻³. The mixture was vigorously stirred for 10 seconds and then the changes of the silver particle dispersion characteristics were measured using the following methods: DLS, UV-Vis absorption spectroscopy, and TEM. The course of the particle size changes was similar to the data presented in Example 1 (Fig 3). However, after 15 minutes following the addition of chloride anions, the final particle size determined by DLS method was 250 nm.

### Example 5

### Recrystallization of silver nanoparticles with an average size of 25 nm using sodium chloride solution with a concentration of 4 mol.dm⁻³

0.8 ml of a dispersion of silver nanoparticles with the average size of 25 nm and Ag concentration of 0.001 mol.dm⁻³ in the dispersion was transferred into a 3 ml cuvette. Then 0.1 ml of distilled water was added. After stirring the mixture, 0.1 ml of NaCl solution with the concentration of 4 mol.dm⁻³ was added. The final concentration of sodium chloride in the solution was 0.4 mol.dm⁻³. The mixture was vigorously stirred for 10 seconds and then the changes of the silver particle dispersion characteristics were measured using the following methods: DLS, UV-Vis absorption spectroscopy, and TEM. The course of the particle size changes was similar to the data presented in Example 1 (Fig 3). However, after 20 minutes following the addition of chloride anions, the final particle size determined by DLS method was 700 nm.

### Example 6

### Activation of silver nanoparticles with an average size of 25 nm using sodium chloride solution with a concentration of 4 mol.dm⁻³ for the purpose of surface enhanced Raman spectroscopy (with a laser excitation wavelength of 1064 nm)

0.2 ml of a dispersion of silver nanoparticles with the average size of 25 nm and Ag concentration of 0.001 mol.dm⁻³ in the dispersion was transferred into a 3 ml cuvette. Then 0.7 ml of distilled water was added. After stirring the mixture, 0.1 ml of NaCl solution with the concentration of 4 mol.dm⁻³ was added. The final concentration of sodium chloride in the solution was 0.4 mol.dm⁻³. The mixture was vigorously stirred for 10 seconds and then 10 µl of adenine solution (or alternatively another analyzed or examined substance) with the concentration of 0.001 mol.dm⁻³ was added to the mixture. Surface enhanced Raman spectrum was recorded after 15 minutes following the sodium chloride solution addition (Fig. 6). For the evaluation of enhancement, the Raman spectrum of adenine solution (concentration equal to 0.1 mol.dm⁻³) was measured without the presence of activated silver particles. The enhancement factor was determined by comparing the intensities of the most intensive peak in spectrum of adenine at 734 cm⁻¹ and it was estimated to approx. 10⁵.

### Example 7

### Activation of silver nanoparticles with an average size of 25 nm using potassium chloride solution with a concentration of 4 mol.dm⁻³ for the purpose of surface enhanced Roman spectroscopy (with a laser excitation wavelength of 988 nm)

1 ml of a dispersion of silver nanoparticles with the average size of 25 nm and Ag concentration of 0.001 mol.dm-³ in the dispersion was transferred into a 10 ml flask. Then 3.5 ml of distilled water was added. After stirring the mixture, 0.5 ml of KCl solution with the concentration of 4 mol.dm⁻³ was added. The final concentration of potassium chloride in the solution was 0.4 mol.dm⁻³. The mixture was vigorously stirred for 10 seconds and then 10 µl of adenine solution (or alternatively another analyzed or examined substance) with the concentration of 0.0005 mol.dm⁻³ was added to the mixture. Surface enhanced Raman spectrum was recorded after 3 minutes following the potassium chloride solution addition. The surface enhanced Raman spectrum of adenine was similar to spectrum presented in Example 6 (Fig 6). The enhancement factor was estimated to approx. 5·10⁵.

### Example 8 (comparative example)

### Activation of silver nanoparticles with an average size of 60 nm using sodium chloride solution with a concentration of 1 mol.dm⁻³ for the purpose of surface enhanced Raman spectroscopy (with a laser excitation wavelength of 514 nm)

1 ml of a dispersion of silver nanoparticles with the average size of 60 nm and Ag concentration of 0.001 mol.dm⁻³ in the dispersion was transferred into a 10 ml flask. Then 3.5 ml of distilled water was added. After stirring the mixture, 0.5 ml of NaCl solution with the concentration of 1 mol.dm⁻³ was added. The final concentration of sodium chloride in the solution was 0.1 mol.dm⁻³. The mixture was vigorously stirred for 10 seconds and then 10 µl of adenine solution (or alternatively another analyzed or examined substance) with the concentration of 0.001 mol.dm⁻³ was added to the mixture. Surface enhanced Raman spectrum was recorded after 15 minutes following the sodium chloride solution addition. The surface enhanced Raman spectrum of adenine was similar to spectrum presented in Example 6 (Fig 6). The enhancement factor was estimated to approx. 2·10⁵.

### Example 9 (comparative example)

### Activation of silver nanoparticles with an average size of 200 nm using sodium chloride solution with a concentration of 1 mol. dm⁻³ for the purpose of surface enhanced Raman spectroscopy (with a laser excitation wavelength of 1064 nm)

0.2 ml of a dispersion of silver nanoparticles with the average size of 200 nm and Ag concentration of 0.001 mol.dm⁻³ in the dispersion was transferred into a 3 ml cuvette. Then 0.7 ml of distilled water was added. After stirring the mixture, 0.1 ml of NaCl solution with a concentration of 1 mol.dm⁻³ was added. The final concentration of sodium chloride in the solution was 0.1 mol.dm⁻³. The mixture was vigorously stirred for 10 seconds and then 10 µl of adenine solution (or alternatively another analyzed or examined substance) with the concentration of 0.001 mol.dm⁻³ was added to the mixture. Surface enhanced Raman spectrum was recorded after 10 minutes following the sodium chloride solution addition. The surface enhanced Raman spectrum of adenine was similar to spectrum presented in Example 6 (Fig 6). The enhancement factor was estimated to approx. 5·10⁴.

### Example 10

### Activation of silver nanoparticles with an average size of 25 nm using sodium chloride solution with a concentration of 4 mol.dm⁻³ for the purpose of surface enhanced Raman spectroscopy (width a laser excitation wavelength of 532 nm)

0.2 ml of a dispersion of silver nanoparticles with the average size of 25 nm and Ag concentration of 0.001 mol.dm⁻³ in the dispersion was transferred into a 3 ml cuvette. Then 0.6 ml of distilled water was added. After stirring the mixture, 0.2 ml of NaCl solution with a concentration of 4 mol.dm⁻³ was added. The final concentration of sodium chloride in the solution was 0.8 mol.dm⁻³. The mixture was vigorously stirred for 10 seconds and then 10 µl of adenine solution (or alternatively another analyzed or examined substance) with the concentration of 10⁻⁶ mol.dm⁻³ was added to the mixture. The final concentration of sodium chloride in the solution was 0.8 mol.dm⁻³. Surface enhanced Raman spectrum was recorded after 6 minutes following the sodium chloride solution addition. The surface enhanced Raman spectrum of adenine was similar to spectrum presented in Example 6 (Fig 6). The enhancement factor was estimated to approx. 6·10⁷.

### Example 11

### Activation of silver nanoparticles with an average size of 25 nm using potassium chloride solution with a concentration of 4 mol.dm⁻³ for the purpose of surface enhanced Raman spectroscopy (with a laser excitation wavelength of 633 nm)

1 ml of a dispersion of silver nanoparticles with the average size of 25 nm and Ag concentration of 0.001 mol.dm⁻³ in the dispersion was transferred into a 10 ml flask. Then 3.5 ml of distilled water was added. After stirring the mixture, 0.5 ml of KCl solution with the concentration of 4 mol.dm⁻³ was added. The final concentration of potassium chloride in the solution was 0.4 mol.dm⁻³. The mixture was vigorously stirred for 10 seconds and then 10 µl of adenine solution (or alternatively another analyzed or examined substance) with the concentration of 0.001 mol.dm⁻³ was added to the mixture. Surface enhanced Raman spectrum was recorded after 6 minutes following the potassium chloride solution addition. The surface enhanced Raman spectrum of adenine was similar to spectrum presented in Example 6 (Fig 6). The enhancement factor was estimated to approx. 10⁵.

### Example 12

### Activation of silver nanoparticles with an average size of 25 nm using potassium chloride solution with a concentration of 4 mol.dm⁻³ for the purpose of surface enhanced Raman spectroscopy (with a laser excitation wavelength of 780 nm)

1 ml of a dispersion of silver nanoparticles with the average size of 25 nm and Ag concentration of 0.001 mol.dm⁻³ in the dispersion was transferred into a 10 ml flask. Then 3.5 ml of distilled water was added. After stirring the mixture, 0.5 ml of KCl solution with the concentration of 4 mol.dm⁻³ was added. The final concentration of potassium chloride in the solution was 0.4 mol.dm⁻³. The mixture was vigorously stirred for 10 seconds and then 10 µl of adenine solution (or alternatively another analyzed or examined substance) with the concentration of 0.001 mol.dm⁻³ was added to the mixture. Surface enhanced Raman spectrum was recorded after 6 minutes following the potassium chloride solution addition. The surface enhanced Raman spectrum of adenine was similar to spectrum presented in Example 6 (Fig 6). The enhancement factor was estimated to approx. 8·10⁴.

## Claims

1. A method for activation of aqueous dispersions of silver nanoparticles for purposes of surface enhanced Raman spectroscopy, wherein a solution of chloride ions is added to the aqueous dispersion of silver nanoparticles in the presence of oxygen, **characterized in that** the final chloride ion concentration in the activated dispersion ranges from 0.4 mol.dm⁻³ to 1 mol.dm⁻³.

2. The method according to claim 1, **characterized in that** the final chloride ion concentration in the activated dispersion ranges from 0.4 mol.dm⁻³ to 0.8 mol.dm⁻³.

3. The method according to claim 1, **characterized in that** oxygen is added to the dispersion in the course of the activation.

4. The method according to claim 1, **characterized in that** the initial size of silver nanoparticles in the dispersion before the activation is in the range of from 10 nm to 100 nm.

5. The method according to claim 1, **characterized in that** a water-soluble salt containing the chloride anion is used as the source of chloride ions.

## Patentansprüche

1. Verfahren zur Aktivierung von Wasserdispersionen der Silber-Nanoteilchen zwecks der oberflächenverstärkten Raman-Spektroskopie, wobei die Chloridionen zur Wasserdispersion der Silber-Nanoteilchen in Anwesenheit von Sauerstoff zugegeben werden, **gekennzeichnet dadurch, dass** die Ergebniskonzentration der Chloridionen in der aktivierten Dispersion im Bereich von 0,4 mol.dm⁻³ bis 1 mol.dm⁻³ liegt.

2. Verfahren nach dem Anspruch 1, **gekennzeichnet dadurch, dass** die Ergebniskonzentration der Chloridionen in der aktivierten Dispersion im Bereich von 0,4 mol.dm⁻³ bis 0,8 mol.dm⁻³ liegt.

3. Verfahren nach dem Anspruch 1, **gekennzeichnet dadurch, dass** der Sauerstoff im Laufe der Aktivierung zur Dispersion zugegeben wird.

4. Verfahren nach dem Anspruch 1, **gekennzeichnet dadurch, dass** die Anfangsgrösse der Silber-Nanoteilchen in der Dispersion vor der Aktivierung im Bereich von 10 nm bis 100 nm liegt.

5. Verfahren nach dem Anspruch 1, **gekennzeichnet dadurch, dass** als Chloridionen-Quelle ein wasserlösliches Salz mit Chloridanion-Gehalt eingesetzt wird.

## Revendications

1. Procédé d'activation des dispersions aqueuses de nanoparticules d'argent à être utilisé dans une spectroscopie Raman exaltée par effet de surface, dans lequel la solution des ions chlorure est ajoutée à la dispersion aqueuse de nanoparticules d'argent en présence d'oxygène, **caractérisé en ce que** la concentration résultante des ions chlorure dans la dispersion activée est dans l'intervalle de 0,4 mol.dm⁻³ à 1 mol.dm⁻³.

2. Procédé selon la revendication 1, **caractérisé en ce que** la concentration résultante des ions chlorure dans la dispersion activée est dans l'intervalle de 0,4 mol.dm⁻³ à 0,8 mol.dm⁻³.

3. Procédé selon la revendication 1, **caractérisé en ce que** l' oxygène est ajouté à la dispersion au cours de l'activation.

4. Procédé selon la revendication 1, **caractérisé en ce que** la taille initiale des nanoparticules d'argent dans la dispersion préalablement à l'activation est dans l'intervalle de 10 nm à 100 nm.

5. Procédé selon la revendication 1, **caractérisé en ce que** l'on utilise comme source d'ions chlorure un sel soluble dans l'eau comprenant un anion chlorure.
